# EUROPEAN PATENT SPECIFICATION

(11) **EP 1 119 633 B1**
(45) Date of publication and mention of the grant of the patent: **26.12.2007**
(21) Application number: 99954759.9
(22) Date of filing: 05.10.1999
(51) Int. Cl.: C12N 15/82, C12N 15/53, A01H 5/00

(54) **COMMERCIAL PRODUCTION OF LACCASE IN PLANTS**
KOMMERZIELLE HERSTELLUNG VON LACCASE IN PFLANZEN
PRODUCTION COMMERCIALE DE LA LACCASE EXPRIMEE DANS DES PLANTES

(30) Priority: 05.10.1998 US 103031 P
(43) Date of publication of application: 01.08.2001
(73) Proprietor: GENENCOR INTERNATIONAL, INC., Palo Alto, California 94304 (US)
(72) Inventor: HOOD, Elizabeth, College Station, TX 77845 (US); HOWARD, John, College Station, TX 77845 (US); JILKA, Joseph, College Station, TX 77845 (US)
(74) Representative: Bentham, Andrew
(86) International application number: PCT/US1999/023256
(87) International publication number: WO 2000/020615

(56) References cited:
- WO-A-00/05381
- WO-A-97/09431
- WO-A-97/45549
- WO-A-98/11205
- DE-A- 19 752 666
- US-A- 5 693 506
- US-A- 5 770 418
- ONG E ET AL: "Cloning and sequence analysis of two laccase complementary DNAs from the ligninolytic basidiomycete Trametes versicolor" GENE: AN INTERNATIONAL JOURNAL ON GENES AND GENOMES,GB,ELSEVIER SCIENCE PUBLISHERS, BARKING, vol. 196, no. 1-2, 1 September 1997 (1997-09-01), pages 113-119, XP004126336 ISSN: 0378-1119
- CRESTINI C ET AL: "THE EARLY BIODEGRADATION PATHWAYS OF RESIDUAL KRAFT LIGNIN MODEL COMPOUNDS WITH LACCASE" ISWPC,1997, pages 1-5, XP000863181
- BOURBONNAIS R ET AL: "ENZYMATIC DELIGNIFICATION OF KRAFT PULP USING LACCASE AND A MEDIATOR" TAPPI JOURNAL,US,TECHNICAL ASSOCIATION OF THE PULP & PAPER INDUSTRY. ATLANTA, vol. 79, no. 6, 1 June 1996 (1996-06-01), pages 199-204, XP000640089 ISSN: 0734-1415

## Description

### BACKGROUND OF THE INVENTION.

Laccase is an enzyme that is a blue copper oxidase. It is believed to have been first obtained from the Japanese tree *Rhus venicifera*. Yshida, H., Zur Chemie des urushi-Firniss, J. Chem. Soc. (Tokyo) 43, 472, 1883. It has since been found in a number of higher plants, but not at high levels. The main source of laccase for commercial purposes is fungi. White rot fungi *Phanerochaete chrysosporium* and *Trametes (Coriolus) versicolor* are common commercial sources of the enzyme. Other fungi producing laccase include *Polyporus, Pleurotus, Philota, Neurospora, Podospora and Aspergillus*.

The enzyme has a number of uses. Examples include catalyzing the oxidation of compounds such as *o,p*-diphenols, aminophenols, polyphenols, polyamines and inorganic ions. (*See, e*.*g*. Yaropolov et al., "Laccase Properties, Catalytic Mechanism, and Applicability" Applied Biochemistry and Biotechnology 49:257-280 (1994)). Its' use as a marker enzyme in enzyme immunoassay (EIA) has also been discussed, as well as its' use in oxidation of steroids and synthesis of vinblastine, a cytostatic compound used in treating malignant diseases.

The most common use of laccase, however, is in connection with the paper and pulp industry. Lignin is a rigid organic polymer and harsh physicochemical conditions must be used to attack or modify the substance. One answer in the search for means to break down lignin was found in the white rot fungi which can naturally destroy lignin, using laccase. In plants, laccase is localized in woody tissues and cell walls of herbaceous species and it is believed it participates in lignin biosynthesis. It is involved in breaking down lignin as well as creating lignin polymers. It is also especially useful as a "biological glue" when manufacturing glued wood products. Such products include construction and industrial plywood, oriented strand board, particleboard and medium density fiberboard.

Currently, the adhesive used is either a urea-formaldehyde type or a phenolformaldehyde resin. There are disadvantages associated with use of formaldehyde in producing such products. Processing and end use monitoring are required as the levels of formaldehyde cannot exceed certain controls. Thus, there has been considerable interest in using such natural alternatives as laccase. It is reported that more than 1.2 million metric tons of adhesive resin solids are used to bond glued wood products in the United States. Which adhesive is used is driven by cost per unit of production, process compatibility and end-use durability. *See,* "Technical and Market Opportunities for Glued Wood Products" Adhesive Age May 31, 1996 V39, N6 p.609.

An example of such a process is described by Kharazipour et al in U.S. Patent No. 5,505,772 and by Olesen et al. at U.S. Patent 5,618,482. In general, fibers and chips from wood or wood-like materials are defibrated by mechanical, steam, or other process. Laccase is then brought into contact with the material in a solution which may contain various auxiliary elements. Since laccase is a large molecule, a mediator may be utilized to aid the enzyme in penetrating the wood and may be added to the solution. The mix is incubated and may then be shaped into formed boards.

A problem with using laccase produced by white-rot fungi, however, is that it is produced in relatively low amounts. Saloheimo, M. and Niku-Paavola, M-L. "Heterologous Production of a Ligninolytic Enzyme: Expression of the Phlebia radiata Laccase gene in Trichoderma reesei" Bio/Technology 9:987-990 (1991). Native expression is described at about 10,000 U/liter from *Trametes versicolor* after induction, which is about 220-250 mg/liter if all was laccase I, and about 65-75 mg/liter if the laccase was laccase II. Bourbonnais et al. Appl. Environ. Microb. 61, no.5 pp.1876-1880 (May 1995). Other experiments have shown expression in *Myceliophthora thermophila* and in *Aspergillus oryzae* was at about 5 mg/liter. Measurements by the inventors reflect that the natural laccase expression in plants is about less than .001% of soluble plant protein.. Attempts by Saloheimo and Niku-Paavola to improve on these levels by using heterologous expression yielded 20 mg/l secreted active laccase. Berka et al. noted that expression levels are too low for commercial purposes. Berka et al. Applied and Environmental Microbiology p.3151-3157 (Aug. 1997). While others have attempted to introduce laccase-encoding nucleotide sequences int plants for the purpose of changing the lignin content of the plant in WO 98/11205 and WO 97/45549, they do not teach production of laccase at commercially acceptable levels for extraction and use.

WO-A-97/09431 relates to mutants of a blue multi-copper oxidase, comprising (a) a substitution of one or more amino acid residues, (b) and insertion of one or more amino acid residues and/or (c) a deletion of one or more amino acid residues, wherein the substitution, insertion or deletion is carried out at a position which is located no greater than 15Å from a Type 1 (T1) copper site.

US-A-5770418 relates to isolated nucleic acid fragments encoding a fungal oxidoreductase enzyme and the purified enzymes produced thereby. In particular, US-A-5770418 relates to nucleic acid fragments encoding a phenol oxidase, specifically a laccase, of a basidomycote, *Polyporus.*

Ong et al. Gene 196, 113-119 (1997) describes the cloning, sequencing and characterization of cDNAs encoding laccase LccI and a putative LccIV and the gene for LccI from the white-rot basisidomycate *Trametes versicolor.*

The inventors have discovered that it is possible to produce commercially acceptable quantities of laccase in plants. This results in a considerable decrease in cost of producing the enzyme. Thus it is an object of the invention to produce laccase in plants at commercially useful levels.

Finally, also provided are improvements on methods of transforming these and other plants using *Agrobacterium*. Modifications to selection of the bacterial strain used, and of processing the strain are provided.

These and further objectives will become apparent from the description.

### SUMMARY OF THE INVENTION

Plants and a process of using them is described in which commercial levels of laccase are produced in plants.

According to the invention, there is thus provided a transgenic plant comprising a nucleotide sequence encoding laccase, linked to a globulin promoter to effect expression of the laccase in the plant, wherein the laccase is expressed at levels of about 0.01% or higher of the total soluble protein of the plant, wherein the expression of laccase is directed to the seed of the plant by said globulin promoter.

The invention also provides:
- a transgenic seed or a transgenic plant cell of a plant of the invention;
- a method of producing laccase in plants, the method comprising introducing a construct into the plant comprising a nucleotide sequence encoding laccase linked to a globulin promoter which directs expression in the plant such that the laccase is expressed at levels of about 0.01% or higher of the total soluble protein of the plant and wherein the expression of the laccase is preferentially directed to the seed of the plant by said globulin promoter; and
- a method of producing laccase, the method comprising providing biomass from a plurality of plants, of which at least certain plants contain a nucleotide molecule comprising a heterologous nucleotide sequence coding for the laccase, wherein the nucleotide sequence is operably linked to a globulin promoter to effect expression of the laccase by the certain plants, wherein the expression of laccase is produced at levels of about 0.01 % or higher soluble protein in the certain plants and wherein the expression of laccase is directed to the seed of said plant by said globulin promoter, growing the plants to produce a biomass and extracting the laccase from the biomass, thereby to produce laccase.

### DESCRIPTION OF THE DRAWINGS

Figure 1 is p7718, a construct containing the laccase gene driven by the ubiquitin promoter, containing the barley alpha amylase sequence and the maize optimized PAT gene as a selectable marker, driven by 35S promoter. It further contains left and right borders of the t-DNA sequences.
Figure 2 is p7017, a construct which is essentially the same as p7718, except that it also contains the KDEL sequence and a fungal signal sequence.
Figure 3 is p7699, which is essentially the same as p7017, except that the fungal sequence is not present.
Figure 4 is p8908, which is the same as 7718, except it substitutes the globulin promoter for the ubiquitin promoter.

### DETAILED DESCRIPTION OF THE PREFERRED EMBODIMENTS

It has been determined by the inventors that commercial production of laccase in plants is feasible and provides considerable advantages over prior attempts to produce the enzyme from fungi. The level of production in plants according to the invention described herein is at a level which makes it economically advantageous to produce large quantities of the enzyme. Plants are easier to store, more economical to grow, more easily transported and can be far more readily produced in large quantities than can fungi, allowing for even further increases in the amount of enzyme which may be produced.

In accordance with the present invention, a DNA molecule comprising a transformation/expression vector is engineered to incorporate laccase-encoding DNA. Genes encoding this enzyme are well known. Some examples include the gene of laccase I cloned from *Aspergillus nidulans* as reported in Aramayo and Timberlake, Nucleic Acids Res. 18:3415 (1990); a laccase gene from *Phlebia radiata* and *Trichoderma reesei* described by Salohemo and Nicku-Paavola, *supra*; expressed in another fungus; a gene from *Myceliophihora termophila* is discussed by Berka et al, *supra* and expressed in another fungus; a laccase gene from eucalyptus and pine for use in controlling lignin content in the plants is described in PCT/NS97/00112; a laccase-encoding tobacco gene is shown to also be used in controlling lignin content of the transformed plant at WO 97/45549; a laccase-encoding gene corresponding to a *Rhizoctonia solani* gene is set forth in 5,480,801 and expressed in a microbe; and a gene from a basidiomycete, *Polyporus pinsitus* is discussed in U.S. patent 5,667,531, also expressed in a transformed microbe. The gene used in the present invention may be from *Trametes versicolor.* Therefore, a gene for use in the present invention can be subcloned in a vector of choice.

In another example of DNA isolation, it is possible to screen a cDNA library with anti-laccase antibodies. The known methodologies used would include identification of the gene by hybridization with probes, PCR, probe/promoter/synthetic gene synthesis, sequencing, molecular cloning and other techniques which are well known to those skilled in molecular biology. While it is possible to synthesize the gene to reflect preferred codon usage in plants, and may be useful in increasing expression of laccases, (*See*, Murray et al, Nucleic Acid Res. 17:477-498 (1980)), it may not be necessary in all cases, as was found with the gene used in the examples below.

In addition to the exemplified laccase DNA and proteins taught herein, the present invention contemplates the utilization of homologous or substantially identical DNA sequences or proteins. The term "identical" in the context of two polypeptide or nucleic acid sequences refers to the residues in the two sequences that are the same when aligned for maximum correspondence, as measured using one of the following "sequence comparison algorithms." Optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat 'l Acad Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection.

An example of an algorithm that is suitable for determining sequence similarity is the BLAST algorithm, which is described in Altschul, et al., J. Mol. Biol. 215:403-410 (1990). Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence that either match or satisfy some positive-valued threshold score T when aligned with a word of the same length in a database sequence. These initial neighborhood word hits act as starting points to find longer HSPs containing them. The word hits are expanded in both directions along each of the two sequences being compared for as far as the cumulative alignment score can be increased. Extension of the word hits is stopped when: the cumulative alignment score falls off by the quantity X from a maximum achieved value; the cumulative score goes to zero or below; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff & Henikoff, Proc. Natl. Acad. Sci. USA 89:10915 (1989)) alignments (B) of 50, expectation (E) of 10, M'5, N'-4, and a comparison of both strands.

The BLAST algorithm then performs a statistical analysis of the similarity between two sequences (*see, e.g.,* Karlin & Altschul, Proc. Nat'l. Acad Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a cellulase nucleic acid of this invention if the smallest sum probability in a comparison of the test nucleic acid to a cellulase nucleic acid is less than about 0.1, more preferably less than about 0.01, and most preferably less than about 0.001. Where the test nucleic acid encodes a cellulase polypeptide, it is considered similar to a specified cellulase nucleic acid if the comparison results in a smallest sum probability of less than about 0.5, and more preferably less than about 0.2.

Another indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, preferably 90% sequence identity, and most preferably 100% sequence identity (i.e., complementary) with each other.

The terms "stringent conditions" or "stringent hybridization conditions" includes reference to conditions under which a probe will hybridize to its target sequence, to a detectably greater degree than other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (*e*.*g*., 10 to 50 nucleotides) and at least about 60°C for long probes (*e.g.*, greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

Specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 138:267-284 (1984): Tₘ = 81.5 °C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization and/or wash conditions can be adjusted to hybridize to sequences of the desired identity. For example, if sequences with ≥90% identity are sought, the Tₘ can be decreased 10 °C. Generally, stringent conditions are selected to be about 5 °C lower than the thermal melting point (Tₘ) for the specific sequence and its complement at a defined ionic strength and pH. However, severely stringent conditions can utilize a hybridization and/or wash at 1, 2, 3, or 4 °C lower than the thermal melting point (Tₘ); moderately stringent conditions can utilize a hybridization and/or wash at 6, 7, 8, 9, or 10 °C lower than the thermal melting point (Tₘ); low stringency conditions can utilize a hybridization and/or wash at 11, 12, 13, 14, 15, or 20 °C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology--Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995).

In a preferred embodiment of the invention, expression of the enzyme in the plant may be increased by directing expression to the cell wall. This may be accomplished by use of a signal sequence and in a preferred embodiment is the barley alpha amylase signal sequence, Rogers, J. Biol. Chem. 260:3731-3738 (1985), or brazil nut protein signal sequence when used in canola or other dicot. Another alternative is to express the enzyme in the endoplasmic reticulum of the plant cell. This may be accomplished by use of a localization sequence, such as KDEL. This sequence contains the binding site for a receptor in the endoplasmic reticulum. Munro, S. and Pelham, H.R.B. 1987 "A C-terminal signal prevents secretion of luminal ER proteins." Cell. 48:899-907. The use of such a localization sequence will increase expression over levels obtained when the enzyme is otherwise expressed in the cytoplasm.

The methods available for putting together such a relatively short synthetic gene comprising the various modifications for improved expression described above can differ in detail. However, the methods generally include the designing and synthesis of overlapping, complementary synthetic oligonucleotides which are annealed and ligated together to yield a gene with convenient restriction sites for cloning. The methods involved are standard methods for a molecular biologist.

Once the gene has been isolated and engineered to contain some or all of the features described above, it is placed into an expression vector by standard methods. The selection of an appropriate expression vector will depend upon the method of introducing the expression vector into host cells. A typical expression vector contains prokaryotic DNA elements coding for a bacterial replication origin and an antibiotic resistance gene to provide for the growth and selection of the expression vector in the bacterial host; a cloning site for insertion of an exogenous DNA sequence, which in this context would code for the laccase; eukaryotic DNA elements that control initiation of transcription of the exogenous gene, such as a promoter; and DNA elements that control the processing of transcripts, such as transcription termination/polyadenylation sequences. It also can contain such sequences as are needed for the eventual integration of the vector into the plant chromosome.

In a preferred embodiment, the expression vector also contains a gene encoding a selection marker which is functionally linked to a promoter that controls transcription initiation. For a general description of plant expression vectors and reporter genes, see Gruber et al, "Vectors for Plant Transformation" in Methods of Plant Molecular Biology and Biotechnology 89-119 (CRC Press, 1993). According to the invention, a tissue specific promoter is provided to direct transcription of the DNA to the seed. This promoter is the globulin promoter, which is the promoter of the maize globulin-1 gene, described by Belanger, F.C. and Kriz, A.L. at "Molecular Basis for Allelic Polymorphism of the Maize Globulin-1 gene" Genetics 129:863-972 (1991). It also can be found as accession number L22344 L22295 in the Genebank database and is set forth below.

### Globulin promoter

In another preferred embodiment, the selective gene is a glufosinate-resistance encoding DNA and in a preferred embodiment can be the phosphinothricin acetyl transferase ("PAT") or maize optimized PAT gene under the control of the CaMV 35S promoter. The gene confers resistance to bialaphos. *See,* Gordon-Kamm et al, The Plant Cell 2:603 (1990); Uchimiya et al, Bio/Technology 11:835 (1993), and Anzai et al, Mol. Gen. Gen. 219:492 (1989).

Obviously, many variations on the selectable markers and other components of the construct are available to one skilled in the art.

In accordance with the present invention, a transgenic plant is produced that contains a DNA molecule, comprised of elements as described above, integrated into its genome so that the plant expresses a heterologous laccase-encoding DNA sequence. In order to create such a transgenic plant, the expression vectors containing the gene can be introduced into protoplasts, into intact tissues, such as immature embryos and meristems, into callus cultures, or into isolated cells. Preferably, expression vectors are introduced into intact tissues. General methods of culturing plant tissues are provided, for example, by Miki et al, "Procedures for Introducing Foreign DNA into Plants" in Methods in Plant Molecular Biology and Biotechnology, Glick et al (eds) pp. 67-68 (CRC Press 1993) and by Phillips et al, "Cell/Tissue Culture and In Vitro Manipulation" in Corn and Corn Improvement 3d Edit. Sprague et al (eds) pp. 345-387 (American Soc. Of Agronomy 1988). The selectable marker incorporated in the DNA molecule allows for selection of transformants.

Methods for introducing expression vectors into plant tissue available to one skilled in the art are varied and will depend on the plant selected. Procedures for transforming a wide variety of plant species are well known and described throughout the literature. *See,* for example, Miki et al, supra; Klein et al, Bio/Technology 10:268 (1992); and Weisinger et al., Ann. Rev. Genet. 22: 421-477 (1988). For example, the DNA construct may be introduced into the genomic DNA of the plant cell using techniques such as microprojectile-mediated delivery, Klein et al., Nature 327: 70-73 (1987); electroporation, Fromm et al., Proc. Natl. Acad Sci. 82: 5824 (1985); polyethylene glycol (PEG) precipitation, Paszkowski et al., Embo J. 3: 2717-2722 (1984); direct gene transfer, WO 85/01856 and EP No. 0 275 069; in vitro protoplast transformation, U.S. Patent No. 4,684,611; and microinjection of plant cell protoplasts or embryogenic callus. Crossway, Mol. Gen. Genetics 202:179-185 (1985). Cocultivation of plant tissue with *Agrobacterium tumefaciens* is another option, where the DNA constructs are placed into a binary vector system. Ishida et al., "High Efficiency Transformation of Maize (Zea mays L.) Mediated by Agrobacterium tumefaciens" Nature Biotechnology 14:745-750 (1996). The virulence functions of the *Agrobacterium tumefaciens* host will direct the insertion of the construct into the plant cell DNA when the cell is infected by the bacteria. See, for example Horsch et al., Science 233: 496-498 (1984), and Fraley et al., Proc. Natl. Acad Sci. 80: 4803 (1983).

Standard methods for transformation of canola are described by Moloney et al. "High Efficiency Transformation of Brassica napus Using Agrobacterium Vectors" Plant Cell Reports 8:238-242 (1989). Corn transformation is described by Fromm et al, Bio/Technology 8:833 (1990) and Gordon-Kamm et al, *supra. Agrobacterium* is primarily used in dicots, but certain monocots such as maize can be transformed by *Agrobacterium*. U.S. Patent No. 5,550,318. Rice transformation is described by Hiei et al., "Efficient Transformation of Rice (Oryza sativs L.) Mediated by Agrobacterium and Sequence Analysis of the Boundaries of the T-DNA" The Plant Journal 6(2): 271-282 (1994), Christou et al, Trends in Biotechnology 10:239 (1992) and Lee et al, Proc. Nat'l Acad. Sci. USA 88:6389 (1991). Wheat can be transformed by techniques similar to those used for transforming corn or rice. Sorghum transformation is described by Casas et al, supra and by Wan et al, Plant Physiolog. 104:37 (1994). Soybean transformation is described in a number of publications, including U.S. Patent No. 5,015,580.

In one preferred method, the *Agrobacterium* transformation methods of Ishida *supra* and also described in U.S. Patent 5,591,616, are generally followed, with modifications that the inventors have found improve the number of transformants obtained. The Ishida method uses the A188 variety of maize that produces Type I callus in culture. In one preferred embodiment the High II maize line is used which initiates Type II embryogenic callus in culture. While Ishida recommends selection on phosphinothricin when using the *bar* or PAT gene for selection, another preferred embodiment provides for use of bialaphos instead.

The bacterial strain used in the Ishida protocol is LBA4404 with the 40kb super binary plasmid containing three vir loci from the hypervirulent A281 strain. The plasmid has resistance to tetracycline. The cloning vector cointegrates with the super binary plasmid. Since the cloning vector has an *E. coli* specific replication origin, it cannot survive in *Agrobacterium* without cointegrating with the super binary plasmid. Since the LBA4404 strain is not highly virulent, and has limited application without the super binary plasmid, the inventors have found in yet another embodiment that the EHA101 strain is preferred. It is a disarmed helper strain derived from the hypervirulent A281 strain. The cointegrated super binary/cloning vector from the LBA4404 parent is isolated and electroporated into EHA 101, selecting for spectinomycin resistance. The plasmid is isolated to assure that the EHA101 contains the plasmid.

Further, the Ishida protocol as described provides for growing fresh culture of the *Agrobacterium* on plates, scraping the bacteria from the plates, and resuspending in the co-culture medium as stated in the '616 patent for incubation with the maize embryos. This medium includes 4.3g MS salts, 0.5 mg nicotinic acid, 0.5 mg pyridoxine hydrochloride, 1.0ml thiamine hydrochloride, casamino acids, 1.5 mg 2,4-D, 68.5g sucrose and 36g glucose, all at a pH of 5.8.In a further preferred method, the bacteria are grown overnight in a 1ml culture, then a fresh 10 ml culture re-inoculated the next day when transformation is to occur. The bacteria grow into log phase, and are harvested at a density of no more than OD600 = 0.5 and is preferably between 0.2 and 0.5. The bacteria are then centrifuged to remove the media and resuspended in the co-culture medium. Since Hi II is used, medium preferred for Hi II is used. This medium is described in considerable detail by Armstrong, C.I. and Green C.E. "Establishment and maintenance of friable, embryogenic maize callus and involvement of L-proline" Planta (1985) 154:207-214. The resuspension medium is the same as that described above. All further Hi II media are as described in Armstrong et al. The result is redifferentiation of the plant cells and regeneration into a plant. Redifferentiation is sometimes referred to as dedifferentiation, but the former term more accurately describes the process where the cell begins with a form and identity, is placed on a medium in which it loses that identity, and becomes "reprogrammed" to have a new identity. Thus the scutellum cells become embryogenic callus.

It is preferred to select the highest level of expression of laccase, and it is thus useful to ascertain expression levels in transformed plant cells, transgenic plants and tissue specific expression. One such method is an ELISA assay which uses biotinylated anti-laccase polyclonal antibodies and an alkaline phosphatase conjugate. For example, an ELISA used for quantitative determination of laccase levels can be an antibody sandwich assay, which utilizes polyclonal rabbit antibodies obtained commercially. The antibody is conjugated to alkaline phosphatases for detection.

The levels of expression of the gene of interest can be enhanced by the stable maintenance of a laccase encoding gene on a chromosome of the transgenic plant. Use of linked genes, with herbicide resistance in physical proximity to the laccase gene, would allow for maintaining selective pressure on the transgenic plant population and for those plants where the genes of interest are not lost.

With transgenic plants according to the present invention, laccase can be produced in commercial quantities. Thus, the selection and propagation techniques described above yield a plurality of transgenic plants which are harvested in a conventional manner. The plant with the laccase can be used in the processing, or the laccase extracted. When using the plant itself, it can, for example, be powdered and then applied in the commercial process, or the seed made into flour. Laccase extraction from biomass can be accomplished by known methods which are discussed, for example, by Heney and Orr, Anal. Biochem. 114: 92-96 (1981).

It is evident to one skilled in the art that there can be loss of material in any extraction method used. Thus, a minimum level of expression is required for the process to be economically feasible. For the relatively small number of transgenic plants that show higher levels of expression, a genetic map can be generated, via conventional RFLP and PCR analysis, which identifies the approximate chromosomal location of the integrated DNA molecule. For exemplary methodologies in this regard, see Glick and Thompson, in Methods in Plant Molecular Biology and Biotechnology 269-84 (CRC Press 1993). Genetic mapping can be effected, first to identify DNA fragments which contain the integrated DNA and then to locate the integration site more precisely. This further analysis would consist primarily of DNA hybridizations, subcloning and sequencing. The information thus obtained would allow for the cloning of a corresponding DNA fragment from a plant not engineered with a heterologous laccase gene. ( Here, "corresponding" refers to a DNA fragment that hybridizes under stringent conditions to the fragment containing the laccase encoding gene). The cloned fragment can be used for high level expression of another gene of interest. This is accomplished by introducing the other gene into the plant chromosome, at a position and in an orientation corresponding to that of the heterologous gene. The insertion site for the gene of interest would not necessarily have to be precisely the same as that of the laccase gene, but simply in near proximity. Integration of an expression vector constructed as described above, into the plant chromosome then would be accomplished via recombination between the cloned plant DNA fragment and the chromosome. Recombinants, where the gene of interest resides on the chromosome in a position corresponding to that of the highly expressed laccase gene likewise should express the gene at high levels.

The laccase gene may also be included in a plant that contains one of the mediators that is useful in commercial application of laccase. Laccase is a large molecule, and hence when used in such processes as delignification, may be enhanced by the use of a mediator. It was found that fungi that degrade wood secrete low molecular weight compounds which act to allow penetration of the wood fibers. Mediators include 1-hydroxybenzotriazole (HBT), 2,2' azeno-bis-(3-ethylbenzothiazoline-6-sulfonic acid) (ABTS), 1-nitroso-2-naphthol-3,6-disulfonic acid (NNS) and chlorpromazine (CPZ), to name a few. One review of such mediators is found at M. Amann, "The Lignozyme Process-Coming Closer to the Mill" *International Symposium on Wood and Pulping Chemistry* 9^{th}, Montreal, Technical Section, Canadian Pulp and Paper Association. Classes of enhancers are also described in WO 94/12619, WO 94/12620, WO 94/12621 and WO 98/23716. An overview of laccase and mediators is provided at H.P. Call, I. Mucke, Journal of Biotechnology (1997) 53:163-202.

Any method to combine the gene with the plant having the mediator will meet the goal, and the process used will vary depending upon the resources of the manufacturer, and the plants involved. By way of example, the laccase gene may be introduced directly into the plant, or a plant with the laccase gene may be backcrossed into the plant having the mediator substance. Backcrossing is a method in which a desirable trait is transferred from one plant into another plant which lacks the trait, but contains other desirable characteristics. By use of a selectable marker, presence of the laccase gene may be confirmed. The progeny is then crossed again to a plant having the mediator. This plant may be then used for generation of laccase and the mediator within the resulting plant, or further backcrossing may be used so that the resulting plant is like the mediator-containing plant in all aspects except that it contains the laccase gene.

The enzyme can be used in a number of different industrial processes. Examples include its use for an in-situ depolymerization of lignin in Kraft pulp, thereby producing a pulp with lower lignin content. Such methods are described in, for example, Jin et al, Holzforschung 45(6): 467-468, 1991; U.S. Patent number 4,432,921. This use of laccase is an improvement over the current use of chlorine for depolymerization of lignin, which leads to the production of chlorinated aromatic compounds, which are an environmentally undesirable by-product of paper mills. Such uses are described in, for example, Current Opinion in Biotechnology 3:261-266, 1992; J. Biotechnol. 25:333-339, 1992, Hiroi et al, Svensk papperstidning 5:162-166, 1976. Production of fibreboard is also possible using laccase, by adding it to a slurry of lignin-containing wood fiber material, forming a mat of the wood fiber material and pressing it by applying heat and pressure. See e.g. U.S. patent number 5,618,482. As mentioned, *supra*, laccase can also substitute for less desirable adhesive resin solids to bond glued wood products. See *Adhesives Age, supra*. Its use as an adhesive includes construction and industrial plywood, oriented strand board, particleboard used for interior applications and medium density fiberboard. These are but a few of the many uses to which the enzyme may be put.

The following illustrates, but is not intended to limit the scope of the invention. It will be evident to one skilled in the art that variations and modifications are possible and fall within the scope and spirit of the invention.

Seed from Hi-II maize kernels were transformed with constructs comprising elements according to the present invention. The constructs are designated p7699, p7718, and p7017. The p7017 construct comprises the ubiquitin promoter, including the first exon and intron; the barley alpha amylase export signal sequence as well as the native fungal signal sequences; KDEL sequence; a laccase-encoding sequence; and the 35S promoter and terminator with the moPAT (maize optimized PAT selectable marker). The p7699 construct is essentially the same as p7017, but does not contain the fungal signal sequence. Construct p7718 contains the barley alpha amylase sequence, but not the fungal signal sequence nor KDEL.

The following provides further detail and is presented by way of illustration and is not intended to limit the scope or spirit of the invention.

### EXAMPLE 1

### Isolation and cloning of laccase encoding DNA

The gene for laccase was cloned from *Trametes versicolor* by the methods described here, with isolated RNA reverse transcribed into cDNA. The sequence is set forth below

### EXAMPLE 2

### Preparation of plasmids

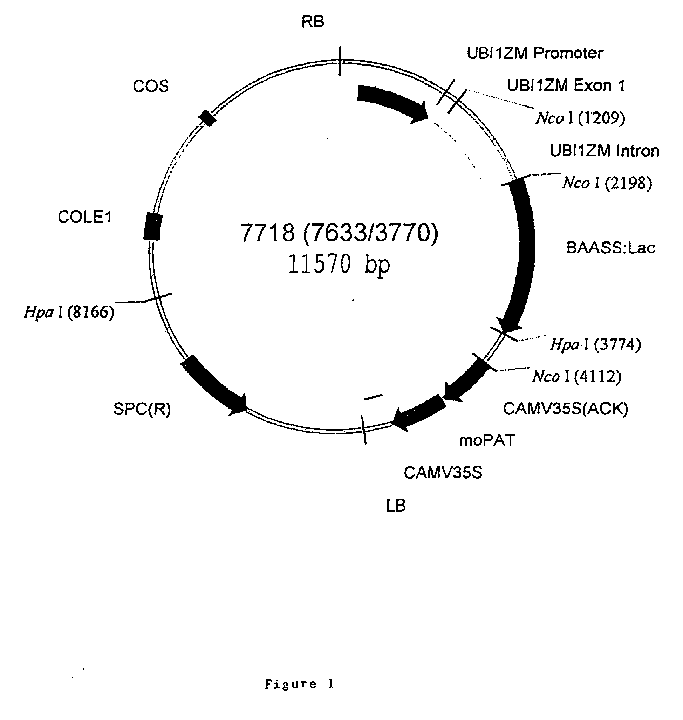

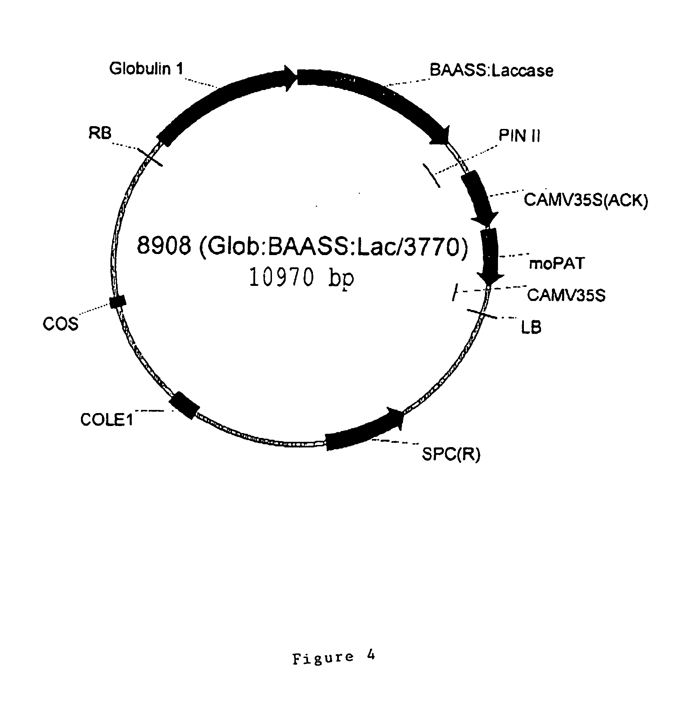

The plasmids containing the barley alpha amylase signal sequences were produced by ligating oligomeric sequences encoding the sequence to the 5' end of the laccase gene, then the entire sequence amplified by PCR and cloned into a pCR® - TOPO vector, available from Invitrogen^{™}. This vector is designed for cloning of TAQ amplified products, and has 3' T overhangs for direct ligation to TAQ amplified PCR products. Since TAQ adds extra A sequence to the 3' end, it links with the overhang in the vector. See e.g. Patent No. 5,487,993. The sequencing of individual clones followed and confirmed the presence of the construct. An individual clone was chosen for further manipulations. To generate plasmid 7718 (Figure 1) intermediate vectors with BAASS:: laccase were cut with NcoI and HpaI and ligated into vector 2774, which contains the ubiquitin promoter and PinII terminator. The entire transcription unit was cut from 2774 with NheI and NotI and ligated to 3770 containing the 35S promoter with the PAT selectable marker between the left and right borders of the *Agrobacterium tumefaciens* gene. For plasmid 8908 (Figure 4) the same procedure was employed, and the ubiquitin promoter of the 2774 vector removed, substituting the globulin promoter. The globulin promoter in p3303 was cut with HindIII and NcoI, and vector 2774 having the ubiquitin, barley alpha amylase, laccase and PinII sequences was cut with the same restriction enzymes. The two pieces were then ligated to create plasmid KB254. While there are several approaches possible for preparing the plasmid, in this procedure the HindIII and NarI site from KB254 was used to cut p7718 and substitute the globulin promoter for the ubiquitin promoter in 7718.

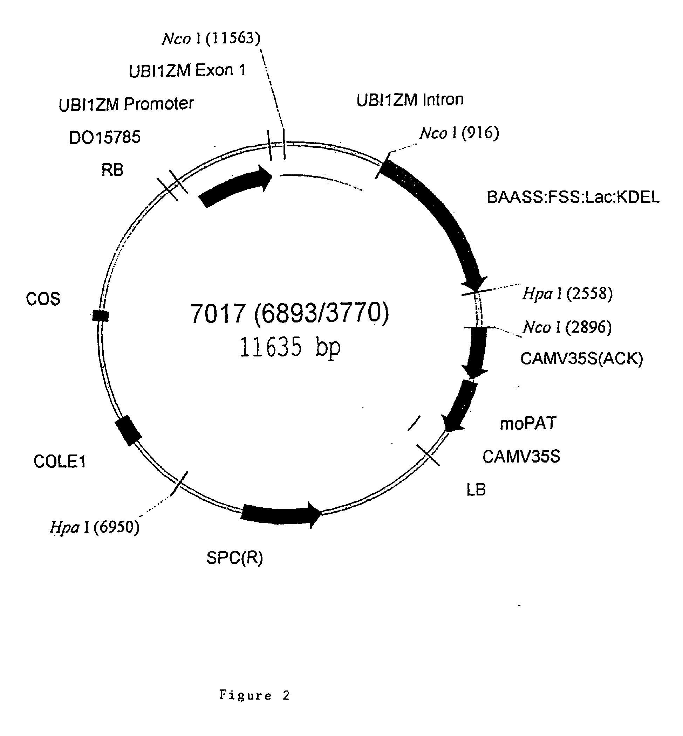

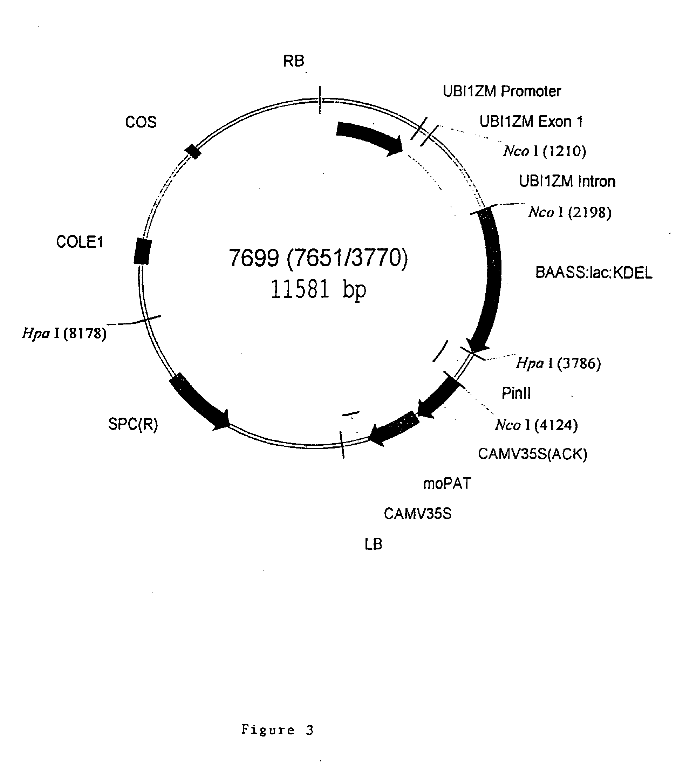

For plasmids 7017 and 7699, (Figures 2 and 3) containing the KDEL sequence, the nucleotides for the amino acids lysine, aspartic acid, glutamic acid and leucine (KDEL) were added to the 3' end of the laccase gene by PCR amplification using a reverse primer containing the KDEL sequence. The entire coding sequence is then put into 2774 containing the ubiquitin promoter and the PinII terminator. Following this it is cut with Nhel and NotI and ligated to 3770 as described above, to generate 7017 and 7699.

### EXAMPLE 3

### Transformation of Maize

Fresh immature zygotic embryos were harvested from Hi-II maize kernels at 1-2 mm in length. The general methods of *Agrobacterium* transformation were used as described by Japan Tobacco, at lshida, Y, H Saito, S Ohta, Y Hiei, T Komari and T Kumashiro. 1996. "High efficiency transformation of maize (Zea mays L.) mediated by Agrobacterium tumefaciens" Nature Biotechnology 14:745-750 with the modifications described *supra*. Fresh embryos were treated with 0.5 ml log phase *Agrobacterium* strains EHA 101. Bacteria were grown overnight in a rich medium with kanamycin and spectinomycin to an optical density of 0.5 at 600 nm, pelleted, then re-inoculated in a fresh 10ml culture. The bacteria were allowed to grow into log phase and were harvested at no more dense than OD600=0.5. The bacterial culture is resuspended in a co-culture medium. For transient expression assays, embryos (5-1 0 per tube) were sonicated in the presence of the bacteria for 30 sec (Trick H and J Finer. 1997. "SAAT: sonication-assisted Agrobacterium-mediated transformation." Transgenic Research 6-.329-336), then plated on a solid medium as above. Embryos and bacteria were co-cultivated for 5 days.

For stable transformations, embryos not subjected to sonication were transferred to a bialaphos selective agent on embryogenic callus medium and transferred thereafter every two weeks to allow growth of transformed type II callus. Plants were regenerated from the callus.

### EXAMPLE 4

### Detection of Expression of Laccase

The corn tissue was analyzed by a laccase activity assay and stable and transient expression of laccase confirmed.

Stable expression has been confirmed in p7718 and p7017. Most of the plants of p7699 died for unknown reasons. Assays described below confirmed expression of laccase at levels of 0.1% total soluble protein for plants with p7718 and levels of 0.01% total soluble protein for plants with p7017. See the table below for expression levels.

| **Plasmid** | **Expression in callus^{a}** | **Expression in leaf** | **Expression in T1 seed** |
|---|---|---|---|
| p7017 | (not done) | 0.0007 | 0.044^{b} |
| | | | (0.025) |
| p7699 | 0.01 | 0.085 | 0.011^{c} |
| p7718 | 0.018 | 0.1 | 0.08^{b} |
| | | | (0.055) |
| p8908 | (not done) | (not done) | 0.14^{b} |
| | | | (0.12) |

| | | | |
|---|---|---|---|
| a) Expression levels are shown as percent total soluble protein b) Average of top four seeds (average of top seven seeds) c) Average of top three seeds, only one low expressing event survived. | | | |

The laccase activity assay uses one of the mediators, ABTS (2,2-azino-bis-(3-ethylbenzthiazoline-6-sulfonic acid)) and can use a smaller or larger sample which increases the detectable amounts 100X. In this procedure, 0.2 ml of ABTS was introduced into a cuvette and 1.5 ml of NaOAc at a pH of 5.0 added. One blanks the cuvette, then 0.1 ml of the enzyme sample is added and mixed. Using a spectrophotometer, the change of absorption was measured at 420nm, every 24 hours for 4 days. One ABTS unit is defined as a change of A420 per minute/2 provided the sample is not diluted.

The Enzyme Linked Immunosorbent Assay is performed on corn using anti-laccase polyclonal antibodies and alkaline phosphatase. The seed extracts are combined with buffering solution. After centrifugation and decanting, total protein concentration is assayed and adjusted to one concentration with PBST (phosphate buffered saline with 0.05% v/v polyoxyethylenesorbitan monolaureate (Tween-20)). Anti-laccase antibody-coated plates are used to capture laccase overnight at 4° C. Laccase standards are prepared and added to the wells along-side the test extracts. The plates are washed and diluted with biotinylated anti-laccase antibodies diluted with PBST. Following incubation, the plate is washed and streptavidin-alkaline phosphatase conjugate diluted with PBST is added and incubated. The plate is washed and pNPP substrate solution added and incubated. The plate is read and amount of target protein calculated by interpolation from the standard curve.

Details of the procedures used in these experiments is as follows. Nunc MaxiSorp 96-well microtiter plates, and an absorbance microplate reader were used Coating antibodies used include purified IgG from rabbit; 0.05 M carbonate/bicarbonate coating buffer at pH 9.6; and PBST (phosphate-buffered saline with 0.05% Tween 20). The protein containing plant extract is standardized to 300 ng/µl. Additional materials included biotinylated rabbit secondary antibody; streptavidin-alkaline phosphatase (Jackson cat. No. 016-050-084); substrate buffer, pH 9.8; and pNpp substrate tablets.

The plates were coated by diluting the coating antibody 1: 500 in the coating buffer. Protein was added at 100/ul per well, the plates covered and placed overnight at 4°C on a flat surface. The next day, the plate was washed four times with PBST, patted dry and samples and standards added. To produce enough standards for four plates, the standard was diluted 1:100 in PBST (2 µl and 198 µl PBST) to get 3 ng/ul (X) as set forth below. 100 ul of each standard was added into wells in triplicate.

| | | |
|---|---|---|
| A | 3 ng | 30 µl of X, 2970 µl PBST |
| B | 1 ng | 750 µl of A, 1725 µl PBST |
| C | 0.3 ng | 200 µl of A, 1800 µl PBST |
| D | 0.1 ng | 200 µl of B, 1800 µl PBST |
| E | 0.06 ng | 125 µl of A, 1960 µl PBST |
| F | 0.03 ng | 200 µl of A, 1800 µl PBST |
| G | 0.01 ng | 200 µl of B, 1800 µl PBST |

Next, 90 µl of PBST was loaded to blank and sample wells. Up to 1.0 ug total protein was added for samples without signifiant interference with the standard curve. If more than 1.0 µg load was necessary, the standard curve was spiked with an identical amount of negative control corn seed protein. 10 µl PBST was loaded for buffer blank. A negative control corn seed extract was included on each plate. The plate was covered and incubated at 4°C overnight on a flat surface.

The next day, the plate was washed four times with PBST and patted dry. A secondary antibody was diluted 1:10,000 in PBST. This was added at amounts of 100 ul per well, the wells covered and plates incubated at 37°C for one hour. The plates were washed four times with PBST and patted dry. Streptavidin-alkaline phosphatase was diluted 1:50,000 in PBST. This was added at levels of 100 ul per well, covered and the plates incubated at 37°C for one hour. The plates were washed four times with PBST and patted dry. Alkaline-phosphatase substrate solution was prepared by diluting tablets up to one tablet per 5 ml of substrate buffer. Once dissolved, 100 ul of substrate was added per well, covered and plates incubated at 37°C for 30 minutes. After this time, the results were read at 405 nm on the absorbance microplate reader. The highest standard had OD around 0.8 to 1.1 and the blank ran at around 0.15 to 0.25.

Southern analysis is a well known technique to those skilled in the art. This common procedure involves isolating the plant DNA, cutting with restriction endonucleases and fractionating the cut DNA on an agarose gel to separate the DNA by molecular weight and transferring to nylon membranes. It is then hybridized with the probe fragment which was radioactively labeled with ³²P and washed in an SDS solution. Southern, E., "Detection of a specific sequences among DNA fragments by gel electrophoresis" J. Mol. Biol. 98:503-517 (1975). Northern analysis is also a commonly used technique by those skilled in the art and is similar to Southern analysis except that RNA is isolated and placed on an agarose gel. The RNA is then hybridized with a labeled probe. Potter, E. et al. "Thyrotropin releasing hormone exerts rapid nuclear effects to increase production of the primary prolactin mRNA transcript" Proc. Nat. Acad. Sci. U.S.A. 78:6662-6666 (1981). A Western analysis is a variation of this technique, where instead of isolating DNA, the protein of interest is isolated and placed on an acrylamide gel. The protein is then blotted onto a membrane and contacted with a labeling substance. *See e.g.,* Hood et al. "Commercial Production of Avidin from Transgenic Maize; Characterization of Transformants, Production, Processing, Extraction and Purification" Molecular Breeding 3:291-306 (1997).

Expression levels of laccase that are produced that are commercially attractive are as follows. While levels at about 0.01 % are commercially useful, expression levels of 0.1 % total soluble protein would be even more attractive, as it would allow recovery of 100mg protein from 22 pounds of corn, which would cost approximately $1.20 to $2.00 for the processed corn. These figures become more commercially viable as expression levels increase. At levels of 1.0%, 100 mg of protein could be recovered from 2.2 pounds of corn at a cost of about $0.10 to 0.20 for the processed corn, and with levels of 10%, 100 mg of protein could be recovered from 0.22 pounds of corn at a cost of about $0.01-0.02 for the processed corn.

Thus it can be seen the invention accomplishes at least all of its objectives.

## Claims

1. A transgenic plant comprising a nucleotide sequence encoding laccase, linked to a globulin promoter to effect expression of the laccase in the plant, wherein the laccase is expressed at levels of about 0.01% or higher of the total soluble protein of the plant, wherein the expression of laccase is directed to the seed of the plant by said globulin promoter.

2. A plant according to claim 1 wherein the laccase is produced at levels of about 0.1% or higher or about 1% or higher.

3. A plant according to claim 1 or 2 wherein the plant is corn.

4. A plant according to any of the preceding claims further comprising a fungal laccase-producing nucleotide sequence.

5. A plant according to any of the preceding claims wherein the plant is maize and wherein the nucleotide sequence is a *Trametes versicolor* nucleotide sequence.

6. A transgenic seed or a transgenic plant cell of a plant according to any of the preceding claims.

7. A method of producing laccase in plants, the method comprising introducing a construct into the plant comprising a nucleotide sequence encoding laccase linked to a globulin promoter which directs expression in the plant such that the laccase is expressed at levels of about 0.01% or higher of the total soluble protein of the plant and wherein the expression of the laccase is directed to the seed of the plant by said globulin promoter.

8. A method according to claim 8 wherein the construct comprises a signal sequence directing expression of the laccase to the plant cell wall.

9. A method according to claim 7 or 8 further comprising a construct comprising introducing a fungal laccase-producing nucleotide sequence.

10. A method according to any of claims 7 to 9 wherein the plant is maize and wherein the nucleotide sequence is a *Trametes versicolor* nucleotide sequence.

11. A method of producing laccase, the method comprising providing biomass from a plurality of plants, of which at least certain plants contain a nucleotide molecule comprising a heterologous nucleotide sequence coding for the laccase, wherein the nucleotide sequence is operably linked to a globulin promoter to effect expression of the laccase by the certain plants, wherein the laccase is produced at levels of about 0.01 % or higher soluble protein in the certain plants and wherein expression of the laccase is directed to the seed of said plant by said globulin promoter, growing the plants to produce a biomass and extracting the laccase from the biomass, thereby to produce laccase.

12. A transgenic plant according to any one of claims 1 to 5, a seed or a plant cell according to claim 6 or a method according to any one of claims 7 to 11, wherein the transgenic plant, seed or plant cell is a monocotylenous transgenic plant, seed or plant cell.

13. A transgenic plant according to any one of claims 1 to 5 or a method according to claim 11, wherein the laccase expression is directed to the plant cell wall.

## Patentansprüche

1. Transgene Pflanze, die eine Nucleotidsequenz einschließt, die Laccase codiert, die mit einem Globulin-Promotor verknüpft ist, um die Expression der Laccase in der Pflanze zu bewirken, wobei die Laccase auf Niveaus von etwa 0,01 % oder höher des gesamten löslichen Proteins der Pflanze exprimiert ist, wobei die Expression der Laccase durch den Globulin-Promotor auf den Samen der Pflanze gelenkt ist.

2. Pflanze nach Anspruch 1, wobei die Laccase auf Niveaus von etwa 0,1 % oder höher oder etwa 1 % oder höher produziert wird.

3. Pflanze nach Anspruch 1 oder 2, wobei die Pflanze Mais ist.

4. Pflanze nach einem der vorhergehenden Ansprüche, die weiterhin eine Pilz-Laccaseproduzierende Nucleotidsequenz umfasst.

5. Pflanze nach einem der vorhergehenden Ansprüche, wobei die Pflanze Mais ist und wobei die Nucleotidsequenz eine *Trametes versicolor*-Nucleotidsequenz ist.

6. Transgener Samen oder transgene Pflanzenzelle einer Pflanze nach einem der vorhergehenden Ansprüche.

7. Verfahren zur Produktion von Laccase in Pflanzen, wobei das Verfahren das Einbringen eines Konstrukts in die Pflanze umfasst, einschließlich einer Nucleotidsequenz, die Laccase codiert, die mit einem Globulin-Promotor verknüpft ist, der die Expression in der Pflanze so ausrichtet, dass die Laccase auf Niveaus von etwa 0,01 % oder höher des gesamten löslichen Proteins der Pflanze exprimiert wird, und wobei die Expression der Laccase durch den Globulin-Promotor auf den Samen der Pflanze gelenkt wird.

8. Verfahren nach Anspruch 7, wobei das Konstrukt eine Signalsequenz einschließt, die die Expression der Laccase auf die Pflanzenzellwand lenkt.

9. Verfahren nach Anspruch 7 oder 8, das weiterhin ein Konstrukt einschließt, das das Einbringen einer Pilz-Laccase-produzierenden Nucleotidsequenz umfasst.

10. Verfahren nach einem der Ansprüche 7 bis 9, wobei die Pflanze Mais ist und wobei die Nucleotidsequenz eine *Trametes versicolor*-Nucleotidsequenz ist.

11. Verfahren zur Herstellung von Laccase, wobei das Verfahren die Bereitstellung von Biomasse aus einer Vielzahl von Pflanzen, wovon mindestens bestimmte Pflanzen ein Nucleotidmolekül enthalten, das eine heterologe Nucleotidsequenz einschließt, die die Laccase codiert, wobei die Nucleotidsequenz mit einem Globulin-Promotor operabel verknüpft ist, um die Expression der Laccase durch die bestimmten Pflanzen zu bewirken, wobei die Laccase auf Niveaus von etwa 0,01 % oder höher an löslichem Protein in den bestimmten Pflanzen produziert wird und wobei die Expression der Laccase auf den Samen der Pflanze durch den Globulin-Promotor gelenkt wird; Wachsen lassen der Pflanzen, um eine. Biomasse zu produzieren, und Extrahieren der Laccase aus der Biomasse, um **dadurch** Laccase zu produzieren, umfasst.

12. Transgene Pflanze nach einem der Ansprüche 1 bis 5, ein Samen oder eine Pflanzenzelle nach Anspruch 6 oder ein Verfahren nach einem der Ansprüche 7 bis 11, wobei die transgene Pflanze, der Samen oder die Pflanzenzelle eine monocotyledone transgene Pflanze, Samen oder Pflanzenzelle ist.

13. Transgene Pflanze nach einem der Ansprüche 1 bis 5 oder ein Verfahren nach Anspruch 11, wobei die Laccase-Expression auf die Pflanzenzellwand gelenkt ist.

## Revendications

1. Plante transgénique comprenant une séquence nucléotidique codant pour la laccase, liée à un promoteur des globulines pour assurer l'expression de la laccase dans la plante, où la laccase est exprimée à des niveaux d'environ 0,01 % ou plus par rapport aux protéines solubles totales de la plante, l'expression de la laccase étant dirigée vers la graine de la plante par ledit promoteur de globuline.

2. Plante selon la revendication 1, dans laquelle la laccase est produite à des niveaux d'environ 0,1 % ou plus, ou d'environ 1 % ou plus.

3. Plante selon la revendication 1 ou 2, dans laquelle la plante est une céréale.

4. Plante selon l'une quelconque des revendications précédentes, qui comprend en outre une séquence nucléotidique produisant la laccase fongique.

5. Plante selon l'une quelconque des revendications précédentes, où la plante est le maïs, et la séquence nucléotidique est une séquence nucléotidique de *Trametes versicolor*.

6. Graine transgénique ou cellule végétale transgénique d'une plante selon l'une quelconque des revendications précédentes.

7. Procédé de production de laccase dans des plantes, le procédé comprenant l'introduction d'une construction dans là plante comprenant une séquence nucléotidique codant pour la laccase liée à un promoteur de globuline qui dirige l'expression dans la plante de telle sorte que la laccase soit exprimée à des niveaux d'environ 0,01 % ou plus par rapport aux protéines solubles totales de la plante, l'expression de la laccase étant dirigée vers la graine de la plante par ledit promoteur de globuline.

8. Procédé selon la revendication 7, dans laquelle la construction comprend une séquence signal qui dirige l'expression de la laccase vers la paroi des cellules végétales.

9. Procédé selon la revendication 7 ou 8, qui comprend en outre une construction, comprenant l'introduction d'une séquence nucléotidique produisant la laccase fongique.

10. Procédé selon l'une quelconque des revendications 7 à 9, dans lequel la plante est le maïs, et la séquence nucléotidique est une séquence nucléotidique de *Trametes versicolor.*

11. Procédé de production de laccase, le procédé consistant à mettre à disposition une biomasse provenant d'une pluralité de plantes, dont au moins certaines plantes contiennent une molécule nucléotidique comprenant une séquence nucléotidique hétérologue codant pour la laccase, la séquence nucléotidique étant liée de manière opérationnelle à un promoteur de globuline pour assurer l'expression de la laccase par ces certaines plantes, la laccase étant produite à des niveaux d'environ 0,01 % ou plus par rapport aux protéines solubles se trouvant dans les certaines plantes, l'expression de la laccase étant dirigée vers la graine de ladite plante par ledit promoteur de globuline, la croissance des plantes pour produire une biomasse, et l'extraction de la laccase à partir de la biomasse, dans le but de produire la laccase.

12. Plante transgénique selon l'une quelconque des revendications 1 à 5, graine ou cellule végétale selon la revendication 6 ou procédé selon l'une quelconque des revendications 7 à 11, où la plante transgénique, la graine ou la cellule végétale est une plante transgénique, une graine ou une cellule végétale monocotylédone.

13. Plante transgénique selon l'une quelconque des revendications 1 à 5 ou procédé selon la revendication 11, où l'expression de la laccase est dirigée vers la paroi de la cellule végétale.


### SEQUENCE LISTING

<110> PRODIGENE, INC.
<120> COMMERCIAL PRODUCTION OF LACCASE IN PLANTS
<130> 1015
<140>
   <141>
<150> 60/103,301
   <151> 1998-10-05
<160> 3
<170> PatentIn Ver. 2.0
<210> 1
   <211> 1500
   <212> DNA
   <213> Trametes versicolor
<220>
   <221> CDS
   <222> (1)..(1497)
<400> 1
   gcc atc ggg ccg gtg gcg agc ctc gtc gtc gcg aac gcc ccc gtc tcg    48
   Ala Ile Gly Pro Val Ala Ser Leu Val Val Ala Asn Ala Pro Val Ser
    1               5                  10                  15
   ccc gac ggc ttc ctt cgg gat gcc atc gtg gtc aac ggc gtg gtc cct    96
   Pro Asp Gly Phe Leu Arg Asp Ala Ile Val Val Asn Gly Val Val Pro
               20                  25                  30
   tcc ccg ctc atc acc ggg aag aag gga gac cgc ttc cag ctc aac gtc   144
   Ser Pro Leu Ile Thr Gly Lys Lys Gly Asp Arg Phe Gln Leu Asn Val
           35                  40                  45
   gtc gac acc ttg acc aac cac agc atg ctc aag tcc act agt atc cac   192
   Val Asp Thr Leu Thr Asn His Ser Met Leu Lys Ser Thr Ser Ile His
       50                  55                  60
   tgg cac ggc ttc ttc cag gca ggc acc aac tgg gca gac gga ccc gcg   240
   Trp His Gly Phe Phe Gln Ala Gly Thr Asn Trp Ala Asp Gly Pro Ala
    65                  70                  75                  80
   ttc gtc aac cag tgc cct att gct tcc ggg cat tca ttt ctg tac gac   288
   Phe Val Asn Gln Cys Pro Ile Ala Ser Gly His Ser Phe Leu Tyr Asp
                   85                  90                  95
   ttc cat gtg ccc gac cag gca gga acg ttc tgg tac cac agt cat ctg   336
   Phe His Val Pro Asp Gln Ala Gly Thr Phe Trp Tyr His Ser His Leu
               100                 105                 110
   tct acg caa tac tgt gac ggg ctg cga gga ccg ttc gtc gtg tac gac    384
   Ser Thr Gln Tyr Cys Asp Gly Leu Arg Gly Pro Phe Val Val Tyr Asp
           115                 120                 125
   ccc aag gat ccg cac gcc agc cgc tac gat gtt gac aac gag agc acg    432
   Pro Lys Asp Pro His Ala Ser Arg Tyr Asp Val Asp Asn Glu Ser Thr
       130                 135                 140
   gtc atc acg ttg acc gac tgg tac cac acc gct gcc cgg ctc ggt ccc    480
   Val Ile Thr Leu Thr Asp Trp Tyr His Thr Ala Ala Arg Leu Gly Pro
   145                 150                 155                 160
   agg ttc cca ctc ggc gcg gac gcc acg ctc atc aat ggt ctt ggg cgg    528
   Arg Phe Pro Leu Gly Ala Asp Ala Thr Leu Ile Asn Gly Leu Gly Arg
                   165                 170                 175
   tcg gcc tcc act ccc acc gcc gcg ctt gct gtg atc aac gtc cag cac    576
   Ser Ala Ser Thr Pro Thr Ala Ala Leu Ala Val Ile Asn Val Gln His
               180                 185                 190
   gga aag cgc tac cgc ttc cgt ctc gtt tcg atc tcg tgc gac ccg aac    624
   Gly Lys Arg Tyr Arg Phe Arg Leu Val Ser Ile Ser Cys Asp Pro Asn
           195                 200                 205
   tac acg ttc agc atc gac ggg cac aat ctg acc gtc atc gag gtc gac    672
   Tyr Thr Phe Ser Ile Asp Gly His Asn Leu Thr Val Ile Glu Val Asp
       210                 215                 220
   ggt atc aac agc cag cct ctc ctt gtc gac tct atc cag atc ttc gcc    720
   Gly Ile Asn Ser Gln Pro Leu Leu Val Asp Ser Ile Gln Ile Phe Ala
   225                 230                 235                 240
   gcg cag cgc tac tcc ttt gtg ttg aat gcg aac caa acg gtc ggc aac    768
   Ala Gln Arg Tyr Ser Phe Val Leu Asn Ala Asn Gln Thr Val Gly Asn
                   245                 250                 255
   tac tgg gtc cgc gcg aac ccg aac ttc gga acg gtt ggg ttc gcc ggg    816
   Tyr Trp Val Arg Ala Asn Pro Asn Phe Gly Thr Val Gly Phe Ala Gly
               260                 265                 270
   ggg atc aac tcc gcc atc ctg cgc tac caa ggc gca cca gtc gcc gag    864
   Gly Ile Asn Ser Ala Ile Leu Arg Tyr Gln Gly Ala Pro Val Ala Glu
           275                 280                 285
   ccc act acg acc cag acg acg tcg gtg atc ccg ctt atc gag acg aac    912
   Pro Thr Thr Thr Gln Thr Thr Ser Val Ile Pro Leu Ile Glu Thr Asn
       290                 295                 300
   ttg cac ccc ctc gct cgc atg cct gtg cct ggc agc ccg aca ccc ggg    960
   Leu His Pro Leu Ala Arg Met Pro Val Pro Gly Ser Pro Thr Pro Gly
   305                 310                 315                 320
   ggc gtc gac aag gcg ctc aac ctc gcg ttt aac ttc aac ggc acc aac    1008
   Gly Val Asp Lys Ala Leu Asn Leu Ala Phe Asn Phe Asn Gly Thr Asn
                   325                 330                 335
   ttc ttc atc aac aac gcg act ttc acg ccg ccg acc gtc ccg gta ctc    1056
   Phe Phe Ile Asn Asn Ala Thr Phe Thr Pro Pro Thr Val Pro Val Leu
                   340                 345                 350
   ctc cag att ctg agc ggt gcg cag acc gca caa gac ctg ctc cct gca    1104
   Leu Gln Ile Leu Ser Gly Ala Gln Thr Ala Gln Asp Leu Leu Pro Ala
           355                 360                 365
   ggc tct gtc tac ccg ctc ccg gcc cac tcc acc atc gag atc acg ctg    1152
   Gly Ser Val Tyr Pro Leu Pro Ala His Ser Thr Ile Glu Ile Thr Leu
       370                 375                 380
   ccc gcg acc gcc ttg gcc ccg ggt gca ccg cac ccc ttc cac ctg cac    1200
   Pro Ala Thr Ala Leu Ala Pro Gly Ala Pro His Pro Phe His Leu His
   385                 390                 395                 400
   ggt cac gcc ttc gcg gtc gtt cgc agc gcg ggg agc acc acg tat aac    1248
   Gly His Ala Phe Ala Val Val Arg Ser Ala Gly Ser Thr Thr Tyr Asn
                   405                 410                 415
   tac aac gac ccg atc ttc cgc gac gtc gtg agc acg ggc acg ccc gcc    1296
   Tyr Asn Asp Pro Ile Phe Arg Asp Val Val Ser Thr Gly Thr Pro Ala
               420                 425                 430
   gcg ggc gac aac gtc acg atc cgc ttc cag acg gac aac ccc ggg ccg    1344
   Ala Gly Asp Asn Val Thr Ile Arg Phe Gln Thr Asp Asn Pro Gly Pro
           435                 440                 445
   tgg ttc ctc cac tgc cac atc gac ttc cac ctc gac gcg ggc ttc gcg    1392
   Trp Phe Leu His Cys His Ile Asp Phe His Leu Asp Ala Gly Phe Ala
       450                 455                 460
   atc gtg ttc gca gag gac gtt gcg gac gtg aag gcg gcg aac ccg gtt    1440
   Ile Val Phe Ala Glu Asp Val Ala Asp Val Lys Ala Ala Asn Pro Val
   465                 470                 475                 480
   ccg aag gcg tgg tcg gac ctg tgc ccg atc tac gac ggg ctg agc gag    1488
   Pro Lys Ala Trp Ser Asp Leu Cys Pro Ile Tyr Asp Gly Leu Ser Glu
                   485                 490                 495
   gct aac cag tga                                                    1500
   Ala Asn Gln
<210> 2
   <211> 499
   <212> PRT
   <213> Trametes versicolor
<400> 2
   Ala Ile Gly Pro Val Ala Ser Leu Val Val Ala Asn Ala Pro Val Ser
    1               5                  10                  15
   Pro Asp Gly Phe Leu Arg Asp Ala Ile Val Val Asn Gly Val Val Pro
               20                  25                  30
   Ser Pro Leu Ile Thr Gly Lys Lys Gly Asp Arg Phe Gln Leu Asn Val
           35                  40                  45
   Val Asp Thr Leu Thr Asn His Ser Met Leu Lys Ser Thr Ser Ile His
       50                  55                  60
   Trp His Gly Phe Phe Gln Ala Gly Thr Asn Trp Ala Asp Gly Pro Ala
   65                  70                  75                  80
   Phe Val Asn Gln Cys Pro Ile Ala Ser Gly His Ser Phe Leu Tyr Asp
                   85                  90                  95
   Phe His Val Pro Asp Gln Ala Gly Thr Phe Trp Tyr His Ser His Leu
               100                 105                 110
   Ser Thr Gln Tyr Cys Asp Gly Leu Arg Gly Pro Phe Val Val Tyr Asp
           115                 120                 125
   Pro Lys Asp Pro His Ala Ser Arg Tyr Asp Val Asp Asn Glu Ser Thr
       130                 135                 140
   Val Ile Thr Leu Thr Asp Trp Tyr His Thr Ala Ala Arg Leu Gly Pro
   145                 150                 155                 160
   Arg Phe Pro Leu Gly Ala Asp Ala Thr Leu Ile Asn Gly Leu Gly Arg
                   165                 170                 175
   Ser Ala Ser Thr Pro Thr Ala Ala Leu Ala Val Ile Asn Val Gln His
               180                 185                 190
   Gly Lys Arg Tyr Arg Phe Arg Leu Val Ser Ile Ser Cys Asp Pro Asn
           195                 200                 205
   Tyr Thr Phe Ser Ile Asp Gly His Asn Leu Thr Val Ile Glu Val Asp
       210                 215                 220
   Gly Ile Asn Ser Gln Pro Leu Leu Val Asp Ser Ile Gln Ile Phe Ala
   225                 230                 235                 240
   Ala Gln Arg Tyr Ser Phe Val Leu Asn Ala Asn Gln Thr Val Gly Asn
                   245                 250                 255
   Tyr Trp Val Arg Ala Asn Pro Asn Phe Gly Thr Val Gly Phe Ala Gly
               260                 265                 270
   Gly Ile Asn Ser Ala Ile Leu Arg Tyr Gln Gly Ala Pro Val Ala Glu
           275                 280                 285
   Pro Thr Thr Thr Gln Thr Thr Ser Val Ile Pro Leu Ile Glu Thr Asn
       290                 295                 300
   Leu His Pro Leu Ala Arg Met Pro Val Pro Gly Ser Pro Thr Pro Gly
   305                 310                 315                 320
   Gly Val Asp Lys Ala Leu Asn Leu Ala Phe Asn Phe Asn Gly Thr Asn
                   325                 330                 335
   Phe Phe Ile Asn Asn Ala Thr Phe Thr Pro Pro Thr Val Pro Val Leu
               340                 345                 350
   Leu Gln Ile Leu Ser Gly Ala Gln Thr Ala Gln Asp Leu Leu Pro Ala
   355 360 365
   Gly Ser Val Tyr Pro Leu Pro Ala His Ser Thr Ile Glu Ile Thr Leu
   370 375 380
   Pro Ala Thr Ala Leu Ala Pro Gly Ala Pro His Pro Phe His Leu His
   385 390 395 400
   Gly His Ala Phe Ala Val Val Arg Ser Ala Gly Ser Thr Thr Tyr Asn
   405 410 415
   Tyr Asn Asp Pro Ile Phe Arg Asp Val Val Ser Thr Gly Thr Pro Ala
   420 425 430
   Ala Gly Asp Asn Val Thr Ile Arg Phe Gln Thr Asp Asn Pro Gly Pro
   435 440 445
   Trp Phe Leu His Cys His Ile Asp Phe His Leu Asp Ala Gly Phe Ala
   450 455 460
   Ile Val Phe Ala Glu Asp Val Ala Asp Val Lys Ala Ala Asn Pro Val
   465 470 475 480
   Pro Lys Ala Trp Ser Asp Leu Cys Pro Ile Tyr Asp Gly Leu Ser Glu
   485 490 495
   Ala Asn Gln
<210> 3
   <211> 1401
   <212> DNA
   <213> Zea mays
<220>
   <223> Globulin-1 promoter
<400> 3
   aagcttgccg agtgccatcc ttggacactc gataaagtat attttatttt ttttattttg 60
   ccaaccaaac tttttgtggt atgttcctac actatgtaga tctacatgta ccattttggc 120
   acaattacat atttacaaaa atgttttcta taaatattag atttagttcg tttatttgaa 180
   tttcttcgga aaattcacat ttaaactgca agtcactcga aacatggaaa accgtgcatg 240
   caaaataaat gatatgcatg ttatctagca caagttacga ccgatttcag aagcagacca 300
   gaatcttcaa gcaccatgct cactaaacat gaccgtgaac ttgttatcta gttgtttaaa 360
   aattgtataa aacacaaata aagtcagaaa ttaatgaaac ttgtccacat gtcatgatat 420
   catatataga ggttgtgata aaaatttgat aatgtttcgg taaagttgtg acgtactatg 480
   tgtagaaacc taagtgacct acacataaaa tcatagagtt tcaatgtagt tcactcgaca 540
   aagactttgt caagtgtccg ataaaaagta ctcgacaaag aagccgttgt cgatgtactg 600
   ttcgtcgaga tctctttgtc gagtgtcaca ctaggcaaag tctttacgga gtgtttttca 660
   ggctttgaca ctcggcaaag cgctcgattc cagtagtgac agtaatttgc atcaaaaata 720
   gctgagagat ttaggccccg tttcaatctc acgggataaa gtttagcttc ctgctaaact 780
   ttagctatat gaattgaagt gctaaagttt agtttcaatt accaccatta gctctcctgt 840
   ttagattaca aatggctaaa agtagctaaa aaatagctgc taaagtttat ctcgcgagat 900
   tgaaacaggg ccttaaaatg agtcaactaa tagaccaact aattattagc tattagtcgt 960
   tagcttcttt aatctaagct aaaaccaact aatagcttat ttgttgaatt acaattagct 1020
   caacggaatt ctctgttttt ctaaaaaaaa actgcccctc tcttacagca aattgtccgc 1080
   tgcccgtcgt ccagatacaa tgaacgtacc tagtaggaac tcttttacac gctcggtcgc 1140
   tcgccgcgga tcggagtccc cggaacacga caccactgtg gaacacgaca aagtctgctc 1200
   agaggcggcc acaccctggc gtgcaccgag ccggagcccg gataagcacg gtaaggagag 1260
   tacggcggga cgtggcgacc cgtgtgtctg ctgccacgca gccttcctcc acgtagccgc 1320
   gcggccgcgc cacgtaccag ggcccggcgc tggtataaat gcgcgccacc tccgctttag 1380
   ttctgcatac agccaaccca a                                           1401